# EUROPÄISCHE PATENTSCHRIFT

(11) **EP 0 617 285 B1**
(45) Veröffentlichungstag und Bekanntmachung des Hinweises auf die Patenterteilung: **14.11.2001**
(21) Anmeldenummer: 94104525.4
(22) Anmeldetag: 22.03.1994
(51) Int. Cl.: G01N 33/543, G01N 33/546, G01N 33/53, G01N 33/76

(54) **Verringerung des Hook-Effekts in Immuntests mit teilchenförmigem Trägermaterial**
Decreasing the Hook-effect in immunoassays with carrier particles
Diminuer l'effet d'Hook dans l'immunoessais avec les particules de support

(30) Priorität: 23.03.1993 DE 4309393
(43) Veröffentlichungstag der Anmeldung: 28.09.1994
(73) Patentinhaber: Roche Diagnostics GmbH, 68305 Mannheim (DE)
(72) Erfinder: Burns, Geoffrey, Dr., D-80337 München (DE); Engel, Wolf-Dieter, Dr., D-82340 Feldafing (DE)
(74) Vertreter: Böhm, Brigitte, Dipl.-Chem. Dr.

(56) Entgegenhaltungen:
- EP-A- 0 483 512
- EP-A- 0 572 845
- WO-A-85/02258
- FR-A- 2 652 900

## Beschreibung

Die vorliegende Erfindung betrifft ein Verfahren zur Bestimmung eines bindefähigen Analyten in einer Probeflüssigkeit durch Bindung an einen mit dem Analyten spezifisch bindefähigen Rezeptor, der auf einem in der Probeflüssigkeit befindlichen teilchenförmigen Trägermaterial immobilisiert ist und ein neues Reagenz zur Durchführung eines derartigen Verfahrens.

Immunologische Präzipitationsverfahren, bei denen ein zu bestimmender Analyt mit einem gegen den Analyten gerichteten Bindepartner (z.B. Antikörper) vermischt wird, sind seit langem bekannt. Die durch die immunologische Reaktion hervorgerufene Agglutinierung führt zu Teilchenaggregaten, die sich im Streulichtverhalten von den Ausgangssubstanzen unterscheiden, wodurch eine Bestimmung der Analytenkonzentration ermöglicht wird.

Zur quantitativen Bestimmung des Analyten wird die Lichtstreuung an den Teilchenaggregaten sowohl über die Messung der Streulichtintensität (Nephelometrie) als auch über die Messung des Intensitätsverlustes von durch das Medium tretendem Licht (Turbidimetrie) genutzt. Die Konzentration des Analyten kann unter Verwendung einer Standardkurve quantifiziert werden (C.P. Price and D.H. Newman "Precipitation and Agglutination Methods" in R.F. Massayeff ed., Methods of Immunological Analysis vol. 1, 134-158, VCH Weinheim; D. Riochet "Particle Agglutination" in R.F. Massayeff ed., Methods of Immunological Analysis Vol. 1, 203-214, VCH Weinheim 1993).

Ein grundsätzliches Problem von quantitativen immunologischen Präzipitationsverfahren ergibt sich aus der Form der Reaktionskurve, die ein Maximum bei gleichen Konzentrationen von Analyt und Bindepartner aufweist und bei weiterer Zugabe eines Reaktionspartners wieder abnimmt (Heidelberger Kurve). Dieser Effekt führt dazu, daß einem Meßsignal zwei unterschiedliche Analytenkonzentrationen zugeordnet werden können, was wiederum zu einer fehlerhaften Bestimmung des Analyten führen kann. Dieser Effekt wird als "Hook-Effekt" bezeichnet. Bei vielen Analyten, insbesondere bei Proteinen, liegt die in der Probeflüssigkeit vorkommende Konzentration oft weit jenseits des Maximums der Heidelberger Kurve, so daß eine fehlerhafte Bestimmung aufgrund des Hook-Effekts häufig auftreten kann. Zur Vermeidung dieses Fehlers ist es erforderlich, daß bei der Bestimmung festgestellt wird, ob das Meßsignal sich im aufsteigenden oder absteigenden Ast der Heidelberger Kurve befindet.

Von Schulze und Schwick (Prot.Biol.Fluids 5 (1958), 15-25) wird eine Methode beschrieben, die eine Doppelbestimmung mit zwei verschiedenen Probenverdünnungen vorsieht, so daß im Falle eines Analytenüberschusses bei der stärker verdünnten Probe ein höheres Meßsignal als bei der konzentrierteren Probe gemessen wird.

Tiffany et al. (Clin.Chem. 20 (1974), 1055-1061) beschreiben einen weiteren Zusatz von Antikörpern zur Probeflüssigkeit. Bei Vorliegen eines Antigenüberschusses tritt eine Signalerhöhung ein. Auch durch einen weiteren Zusatz von Antigenmaterial bekannter Konzentration läßt sich ein Antigenüberschuß erkennen (Sternberg, Clin.Chem. 23 (1977), 1456-1464).

Weiterhin sind mehrere Verfahren beschrieben (z.B. DE-A-27 24 722, EP-B-0 148 463), bei denen der Hook-Effekt durch eine aufwendige, rechnergesteuerte Auswertung erkannt werden soll. Es wird versucht, durch Bestimmung der Reaktionsdauer bis zum Auftreten der maximalen Reaktionsgeschwindigkeit bei nephelometrischen Messungen zwischen Antigen- und Antikörperüberschuß zu diskriminieren.

Die oben genannten Verfahren besitzen den Nachteil, daß entweder eine kompliziertere Durchführung der Messung oder eine kompliziertere Auswertung der Messung notwendig ist. Eine bessere Möglichkeit, Fehlinterpretationen bei immunologischen Präzipitationsverfahren des oben genannten Typs zu vermeiden, würde darin bestehen, die Gestalt der Heidelberger Kurve soweit zu verändern, daß das Auftreten des Hook-Effekts ganz vermieden oder in einen Konzentrationsbereich des Analyten verschoben wird, der unter physiologischen Bedingungen nicht mehr auftritt.

In US-A-4,595,661 werden Sandwich- und nephelometrische Immuntests beschrieben, die zur Verringerung des Hook-Effekts neben den normalerweise in einem Immuntest vorhandenen hochspezifischen Antikörpern zusätzlich mindestens einen weiteren Antikörper enthalten, der eine geringe Affinität für den Analyten besitzt. Der Nachteil dieses Verfahrens besteht darin, daß für jeden nachzuweisenden Analyten zwei spezifische Antikörper mit deutlich unterschiedlichen Affinitäten für den Analyten hergestellt werden müssen.

Ein weiteres Verfahren zur Verringerung des Hook-Effekts ist in US-A-4,743,542 beschrieben. Dabei wird ein Sandwich-Test in Gegenwart von markierten und unmarkierten Ligandenbindepartnern (Antikörpern) durchgeführt, wobei durch Kompetition der unmarkierten Ligandenbindepartner mit den markierten Ligandenbindepartnern ein Analytenüberschuß in der Probelösung vermieden wird. Der Nachteil dieses Verfahrens besteht darin, daß die Empfindlichkeit des Tests erheblich verringert wird.

In EP-A-0 483 512 wird offenbart, daß die Zugabe von nichtionischen Polymeren zur Testlösung zu einer Verringerung des Hook-Effekts führen kann.

Zur Erhöhung der Signalstärke bei nephelometrischen oder turbidimetrischen Immuntests ist es weiterhin bekannt, ein teilchenförmiges Trägermaterial (z.B. Latexteilchen oder Goldsole) zu verwenden, die mit einem gegen den zu bestimmenden Analyten gerichteten Bindepartner beschichtet sind. Eine Vermischung der beschichteten Teilchen mit der den Analyten enthaltenden Probe führt bedingt durch das Auftreten von Teilchenaggregaten zu einer quantifizierbaren Änderung des nephelometrischen oder turbidimetrischen Verhaltens der Probeflüssigkeit. Ein derartiger Teilchen-verstärkter Test ist erheblich empfindlicher als ein unverstärkter Test, da weniger Agglutinierungsvorgänge zur Erzeugung eines meßbaren Signals notwendig sind. Auch bei derartigen Teilchen-verstärkten Immuntests besteht jedoch das Problem der Vermeidung und/oder Verminderung des Hook-Effekts.

Eine Aufgabe der vorliegenden Erfindung war es deshalb, ein einfaches immunologisches Präzipitationsverfahren zur Bestimmung eines bindefähigen Analyten in einer Probeflüssigkeit bereitzustellen, bei dem der Hook-Effekt als Störung vermieden und/oder verhindert werden kann.

Die erfindungsgemäße Aufgabe wird durch ein Verfahren zur Bestimmung eines Analyten in einer Probeflüssigkeit durch Bindung des Analyten an einen mit dem Analyten spezifisch bindefähigen Rezeptor R₁, der mit mindestens zwei unterschiedlichen Epitopen auf dem Analyten über R₁ₐ und R_{1b} spezifish bindefähing ist und der auf einem in der Probeflüssigkeit befindlichen teilchenförmigen Trägermaterial immobilisiert ist, gelöst, wobei das Verfahren dadurch gekennzeichnet ist, daß die Probeflüssigkeit weiterhin einen löslichen, mit dem Analyten spezifisch bindefähigen Rezeptor R₂ enthält, der mindestens zwei Bindungsstellen für den Analyten besitzt.

Bei dem erfindungsgemäßen Verfahren enthält die Probeflüssigkeit einen mit dem Analyten spezifisch bindefähigen Rezeptor R₁, der auf einem teilchenförmigen Trägermaterial immobilisiert ist. Der immobilisierte Rezeptor R₁ ist mit mindestens zwei unterschiedlichen Epitopen auf dem Analyten über R₁ₐ und R_{1b}, spezifisch bindefähig. Die Durchführung des Verfahrens kann bei einer derartigen Verwendung von zwei unterschiedlichen R₁ₐ und R_{1b} mit einem Gemisch zweier unterschiedlicher Träger, wobei auf dem ersten Träger R₁ₐ immobilisiert ist und auf dem zweiten Träger. R_{1b} immobilisiert ist, oder mit einem einzigen Träger, auf dem ein Gemisch von R₁ₐ und R_{1b} immobilisiert ist, erfolgen.

Ein bevorzugtes Beispiel für einen immobilisierten Rezeptor R₁ ist das Fab-Fragment eines gegen den zu bestimmenden Analyten gerichteten Antikörpers. Weitere Beispiele für immobilisierte Rezeptoren R₁ sind monoklonale Antikörper, F(ab')₂-und F(ab)₂-Fragmente von Antikörpern, monospezifische polyklonale Antikörper oder auch Antigene oder anti-Idiotyp-Antikörper (wenn der zu bestimmende Analyt ein Antikörper ist) oder andere Rezeptoren, die zu einer hochaffinen spezifischen Bindung mit einem Analyten in der Lage sind (z.B. Streptavidin, Lectine, Protein A etc.). Besonders bevorzugt ist der immobilisierte Rezeptor R₁ ein Gemisch aus mindestens zwei monoklonalen Antikörpern, monospezifischen polyklonalen Antikörpern oder Fragmenten derartiger Antikörper.

Die Erzeuqung von Antikörpern, die gegen unterschiedliche Epitope auf dem gleichen Antigen gerichtet sind, ist in der Literatur ausführlich beschrieben, siehe z.B. Kofler et al., Am.J.Reprod.Immunol. 2 (1982), 212-216; Ehrlich et al., Am.J. Reprod.Immunol. 8 (1985), 48-54; Norman et al., J.Clin.Endocrinol. and Metab. 61 (1985), 1031-1038 und Norman et al., Clin.Chem. (1987), 1147-1151. Allgemein kann man derartige, gegen unterschiedliche Determinanten gerichtete Antikörper durch selektive Immunisierung von Versuchstieren mit spezifischen Antigenfragmenten (z.B. Oligopeptiden) und anschließende Gewinnung der Antikörper aus den Versuchstieren auf bekannte Weise erhalten.

Die vorliegende Erfindung zeichnet sich gegenüber bekannten Verfahren dadurch aus, daß die Probeflüssigkeit weiterhin einen löslichen, mit dem Analyten spezifisch bindefähigen Rezeptor R₂ enthält, der mindestens zwei Bindungsstellen für den Analyten besitzt, d.h. der Rezeptor R₂ ist frei in Lösung und nicht auf den Teilchen des Trägermaterials immobilisiert. Durch Verwendung dieses zusätzlichen Rezeptors R₂ wird eine Verringerung und/oder vollständige Vermeidung des Hook-Effekts bei einem immunologischen Präzipitationsverfahren ermöglicht. Dabei ist es erfindungswesentlich, daß der Rezeptor R₂ mindestens zwei Bindungsstellen für den Analyten besitzt. Bevorzugte Beispiele für den Rezeptor R₂ sind monoklonale Antikörper, monospezifische polyklonale Antikörper oder Antikörperfragmente mit zwei Bindungsstellen für den Analyten (z.B. F(ab)₂, F(ab')₂, aber nicht Fab).

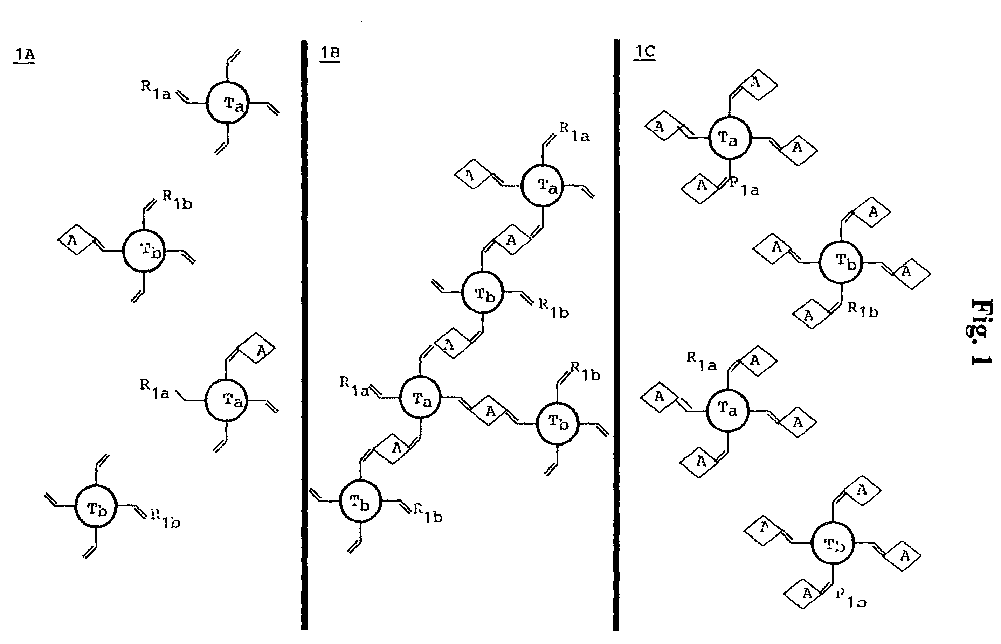

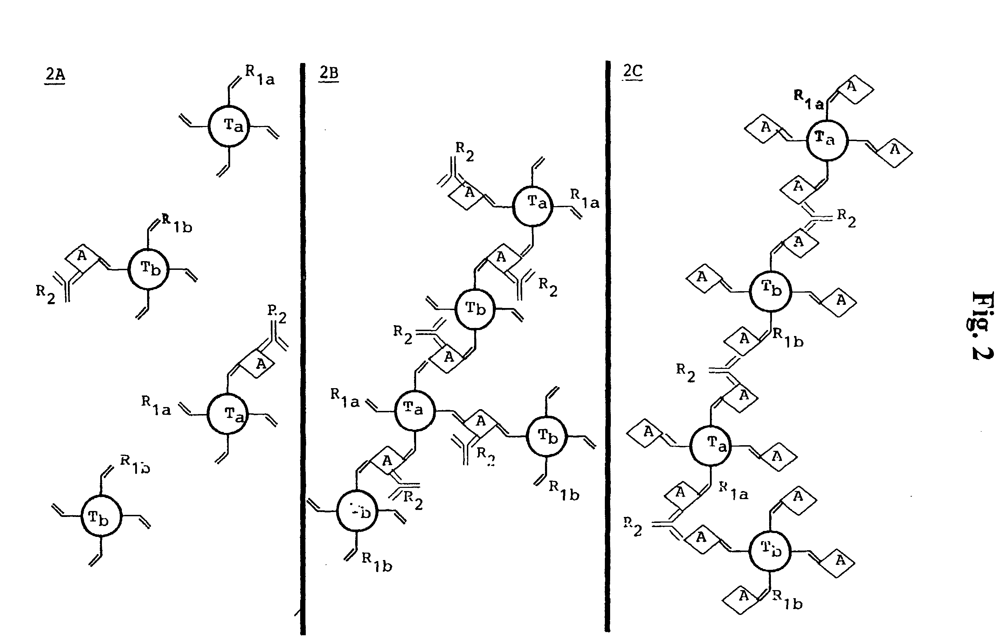

In den Figuren 1 und 2 der beiliegenden Zeichnungen ist der Unterschied zwischen einem immunologischen Präzipitationsverfahren mit immobilisierten R₁ₐ und R_{1b} gemäß dem Stand der Technik (Figur 1) und dem immunologischen Präzipitationsverfahren in Gegenwart eines zusätzlichen löslichen Rezeptors R₂ gemäß vorliegender Erfindung (Figur 2) gezeigt. Figur 1 zeigt die drei Phasen der Heidelberger Kurve, wobei Figur 1A einen Überschuß des Trägermaterials Tₐ, T_{b} mit darauf immobilisierten R₁ₐ, R_{1b} gegenüber dem Analyten A, Figur 1B den Äquivalenzpunkt bei einer gleichen molaren Konzentration des teilchenförmigen Trägermaterials Tₐ, T_{b} und des Analyten A und Figur 1C einen Überschuß des Analyten A zeigt. Das in Figur 1 gezeigte Verfahren gemäß dem Stand der Technik wird mit zwei unterschiedlichen immobilisierten R₁ₐ und R_{1b} durchgeführt, die jeweils auf unterschiedlichen Teilchen Tₐ und T_{b} des Trägermaterials immobilisiert sind. R₁ₐ und R_{1b} erkennen unterschiedliche Epitope auf dem Analyten A. Aus Figur 1A geht hervor, daß bei einem großen Überschuß der Trägertei-lchen Tₐ und T_{b} mit den immobilisierten R₁ₐ und R_{1b} bezüglich des Analyten A ein Trägerteilchen nur maximal ein Molekül des Analyten bindet, so daß in der Probeflüssigkeit nur eine geringe Aggregatbildung (und somit nur ein geringes Meßsignal) auftritt. Bei steigender Analytenkonzentration nimmt diese Aggregation zu, bis die in Figur 1B gezeigte Äquivalenz zwischen Trägerteilchen Tₐ, T_{b} und Analytenmolekülen A erreicht ist. An diesem Äquivalenzpunkt, dem Maximum der Heidelberger Kurve, ist die Aggregation der Teilchen in der Probeflüssigkeit (und damit auch das Meßsignal) am höchsten. Bei einer weiteren Zunahme der Analytenkonzentration findet man aufgrund der zunehmenden Sättigung aller immobilisierten Rezeptorbindestellen eine Abnahme der Aggregation (Figur 1C) und damit eine Abnahme des Meßsignals (Hook-Effekt).

Figur 2 stellt eine Ausführungsform des erfindungsgemäßen Verfahrens dar. Dabei ist neben den Trägerteilchen Tₐ, T_{b} mit den immobilisierten R₁ₐ, R_{1b} und dem Analyten A in der Probeflüssigkeit zusätzlich ein löslicher Rezeptor R₂ mit zwei Bindungsstellen für den Analyten vorhanden. Aus Figur 2A geht hervor, daß bei einem Überschuß der Trägerteilchen gegenüber dem Analyten aufgrund der zusätzlichen Anwesenheit des löslichen Rezeptors R₂ bereits eine etwas stärkere Aggregation als bei dem Verfahren gemäß dem Stand der Technik (Figur 1A) zu beobachten ist. Dies bedeutet eine höhere Empfindlichkeit des erfindungsgemäßen Verfahrens im aufsteigenden Ast der Heidelberger Kurve. Am Äquivalenzpunkt, an dem das molare Verhältnis von Trägerteilchen Tₐ, T_{b} zu Analytenmolekülen A gleich ist, kann die Anwesenheit des zusätzlichen löslichen Rezeptors R₂ eine, die maximale Agglutinierung etwas erhöhende Wirkung besitzen. Bei einem Überschuß des Analyten (Figur 2C), wenn immer mehr immobilisierte R₁ₐ, R_{1b} an ein Analytenmolekül binden, zeigt sich die Wirkung des Zusatzes von R₂ am deutlichsten. Die Gegenwart des zusätzlichen löslichen Rezeptors ermöglicht eine zusätzliche Quervernetzung der Trägerteilchen Tₐ, T_{b} wodurch die bei dem Verfahren des Standes der Technik (Figur 1C) beobachtete Abnahme der Agglutinierung vermieden oder/und verzögert wird.

An dieser Stelle ist anzumerken, daß bei der in Figur 2 gezeigten und bevorzugten Ausführungsform der vorliegenden Erfindung der lösliche Rezeptor R₂ derart ausgewählt ist, daß die Bindung von R₂ an den Analyten nicht mit der Bindung des immobilisierten Rezeptors R₁ an den Analyten interferiert, d.h. der lösliche Rezeptor R, erkennt ein anderes Epitop auf dem Analyten als die immobilisierten R₁ₐ, R_{1b} und die Bindung von R₂ an den Analyten besitzt keinen signifikanten Einfluß auf die Bindung von R₁ₐ und R_{1b} an den Analyten. Bei dieser Ausführungsform der vorliegenden Erfindung erreicht man insbesondere eine etwas höhere Empfindlichkeit im aufsteigenden Ast der Heidelberger Kurve, eine Verschiebung des Maximums zu einer höheren Analytenkonzentration und einen erheblich langsameren Abfall der Heidelberger Kurve bei einer weiteren Zunahme der Analytenkonzentration. Dies führt wiederum zu einem starken Zurückdrängen des Hook-Effekts, da die Analytenkonzentrationen, die jeweils gleiche Signale liefern, weil sie auf dem aufsteigenden oder absteigenden Ast der Heidelberger Kurve liegen, aufgrund der geringeren Abnahme der Agglutinierung bei Analytenüberschuß um ein Vielfaches weiter voneinander entfernt sind.

Die Konzentration des löslichen Rezeptors R₂ in der Probeflüssigkeit hängt natürlich von dem jeweils gewählten Rezeptor, insbesondere von seiner Affinität zum Analyten ab. Es ist jedoch möglich, eine allgemeine Abschätzung für die jeweils günstige Menge durchzuführen.

Bei dem in den Beispielen der vorliegenden Anmeldung beschriebenen Verfahren zum Nachweis von Human-Choriongonadotropin (hCG) liegt der Äquivalenzpunkt des Tests bei etwa 10.000 mIU/ml hCG. An diesem Punkt ist anzunehmen, daß ein Trägerteilchen statistisch ein einziges hCG-Molekül bindet. Um eine weitere Agglutinierung zu ermöglichen, sollte der lösliche Rezeptor R₂ besonders bevorzugt in einer Konzentration zugesetzt werden, die halb so groß wie die Konzentration von hCG in der Probeflüssigkeit ist.

Doch auch kleinere Konzentrationen bringen eine Verbesserung der Agglutinierung und es ist zu erwarten, daß diese vorteilhafte Wirkung bereits bei Konzentrationen des gelösten Rezeptors R₂ in der Probeflüssigkeit von mindestens 5 Mol-% der Konzentration des Analyten am Äquivalenzpunkt auftritt, wobei der Äquivalenzpunkt durch eine gleiche molare Konzentration der Teilchen des Trägermaterials und der Moleküle des Analyten definiert ist.

Vorzugsweise beträgt die Konzentration des löslichen Rezeptors R₂ mindestens 20 Mol-% der Analytenkonzentration am Äquivalenzpunkt und besonders bevorzugt liegt die optimale Konzentration des löslichen Rezeptors R₂ im Bereich von 50 % der Analytenkonzentration am Äquivalenzpunkt plus der zusätzlichen Konzentration an Analyten, die überhalb des Äquivalenzpunkts liegt.

In Zusammenhang mit diesen Abschätzungen der optimalen Konzentration des löslichen Rezeptors R₂ ist jedoch festzustellen, daß diese Konzentrationen sich in der Praxis stark ändern können, so daß beispielsweise bei Verwendung eines löslichen Rezeptors R₂ mit einer geringeren Affinität für den Analyten oder für den über den immobilisierten Rezeptor R₁ an das Trägermaterial gebundenen Analyten die optimale Konzentration von R₂ um mehrere Größenordnungen höher liegen kann.

In einer zweiten Ausführungsform der vorliegenden Erfindung kann man den löslichen Rezeptor R₂ auch derart auswählen, daß die Bindung von R₂ an den Analyten mit der Bindung des immobilisierten Rezeptors R₁ an den Analyten interferiert, d.h. der Rezeptor R₂ erkennt gleiche Epitope auf dem Analyten wie einer der immobilisierten Rezeptoren R₁ oder/und die Bindung des löslichen Rezeptors R₂ an den Analyten beeinträchtigt die Affinität des Analyten zum immobilisierten Rezeptor R₁. Ein Vorteil dieser Ausführungsform der vorliegenden Erfindung besteht darin, daß ein Auftreten des Hook-Effekts vollständig vermieden werden kann, was allerdings auf Kosten der Empfindlichkeit des Tests geht. Diese Ausführungsform kann aber trotzdem für spezielle Aufgaben günstig sein, wenn es weniger auf die Empfindlichkeit des Tests als auf die Vermeidung möglicher Fehlinterpretationen der erhaltenen Meßwerte ankommt. Diese Ausführungsform der vorliegenden Erfindung ist ebenfalls durch ein Beispiel dargestellt.

Für das erfindungsgemäße Verfahren ist es weiterhin bevorzugt, daß die Probeflüssigkeit gemäß der Lehre von EP-B-0 483 512 ein nicht-ionisches Polymer aus der Gruppe, bestehend aus Dextran mit einem Molekulargewicht von mindestens 500 kD, Polyvinylpyrrolidon mit einem Molekulargewicht von mindestens 10 kD und Polyethylenglykol mit einem Molekulargewicht von mindestens 6 kD enthält. Vorzugsweise liegt dieses nichtionische Polymer in einer Konzentration von mindestens 1 Gew.-%, besonders bevorzugt von 2 bis 6 Gew.-% in der Probeflüssigkeit vor. Besonders bevorzugt ist auch die Verwendung eines Polyethylenglykols mit einem Molekulargewicht von 6 kD bis 300 kD, am meisten bevorzugt von 40 kD als nicht-ionisches Polymer.

Das teilchenförmige Trägermaterial, auf dem der Rezeptor R₁ immobilisiert ist, kann ein beliebiges teilchenförmiges Trägermaterial sein, das in der Technik zur Durchführung von Agglutinierungstests bekannt ist. Bevorzugte Beispiele des teilchenförmigen Trägermaterials sind Latexpartikel, Metallsole (z.B. Goldsole, Silbersole oder Eisensole) und Liposomen. Besonders bevorzugte Trägermaterialien sind Latexpartikel und Goldsole. Die Größe der Teilchen reicht vorzugsweise von 10 bis 500 nm, besonders bevorzugt von 50 bis 250 nm.

Die Art der Beschichtung des teilchenförmigen Trägermaterials mit dem Rezeptor R₁ ist ebenfalls nicht kritisch für die Durchführbarkeit der vorliegenden Erfindung. Zu diesem Zweck können alle dem Fachmann auf dem Gebiet von Teilchen-verstärkten Immuntests bekannten Beschichtungsmethoden eingesetzt werden. Ein bevorzugtes Beispiel für die Beschichtung des Trägermaterials mit dem Rezeptor R₁ besteht in der Verwendung von Streptavidin-beschichteten Latexteilchen, die mit biotinylierten Antikörpern oder Antikörperfragmenten überzogen werden.

Unter einem Analyten gemäß vorliegender Erfindung sind Substanzen zu verstehen, die mindestens zwei Epitope, d.h. Bindungsstellen für einen Rezeptor (R₁, R₂) besitzen. Vorzugsweise ist der Analyt ein Protein. Der Analyt kann sich in einer Körperflüssigkeit wie Plasma, Urin, Serum, Speichel oder dgl. oder in einer geeigneten Pufferlösung befinden. Das erfindungsgemäße Verfahren eignet sich besonders z.B. zur Bestimmung von Albumin im Urin, Apolipoprotein A1 und B im Serum oder Plasma, Immunglobulinen, Ferritin, LD(a) oder α-1-Mikroglobulin. Ein besonders bevorzugtes Beispiel ist die Bestimmung von hCG im Serum. Beim Analyten kann es sich auch um einen Antikörper handeln, wobei in diesem Fall als spezifischer Rezeptor das mit dem Antikörper reagierende Antigen oder ein gegen den Antikörper gerichteter Anti-Idiotyp-Antikörper verwendet werden kann.

Die Bestimmung der Analytenkonzentration beim erfindungsgemäßen Verfahren kann mit geeigneten Geräten sowohl nephelometrisch als auch turbidimetrisch durch Vergleich mit einer Standardkurve für bekannte Analytenkonzentrationen erfolgen.

Ein weiterer Gegenstand der vorliegenden Erfindung ist ein Reagenz zur Durchführung eines immunologischen Präzipitationsverfahrens zur Bestimmung eines bindefähigen Analyten in einer Probeflüssigkeit, welches dadurch gekennzeichnet ist, daß es einen an den Analyten spezifisch bindefähigen Rezeptor R₁, der mit mindestens zwei unterschiedlichen Epitopen auf dem Analyten über R₁ₐ und R_{1b} spezifisch bindefähig ist und der auf eine teilchenförmigen Trägermaterial immobilisiert ist, und weiterhin einen mit dem Analyten spezifisch bindefähigen Rezeptor R₂, der in der Probeflüssigkeit löslich ist und mindestens zwei Bindungsstellen für den Analyten besitzt, enthält.

Vorzugsweise enthält das erfindungsgemäße Reagenz weiterhin ein nicht-ionisches Polymer aus der Gruppe, bestehend aus Dextran mit einem Molekulargewicht von mindestens 500 kD, Polyvinylpyrolidon mit einem Molekulargewicht von mindestens 10 kD und Polyethylenglykol mit einem Molekulargewicht von mindestens 6 kD. Das Trägermaterial des erfindungsgemäßen Reagenz ist vorzugsweise aus der Gruppe, bestehend aus Latexpartikeln, Metallsolen und Liposomen ausgewählt, wobei Latexpartikel und Goldsole besonders bevorzugt sind.

Weiterhin bevorzugt ist, daß das erfindungsgemäße Reagenz mindestens zwei unterschiedliche immobilisierte R₁ₐ und R_{1b} enthält, die an unterschiedliche Epitope auf dem Analytenspezifisch bindefähig sind. Besonders bevorzugt ist der immobilisierte Rezeptor R₁ ein Gemisch aus mindestens zwei monoklonalen Antikörpern, monospezifischen polyklonalen Antikörpern oder Fragmenten derartiger Antikörper.

Der lösliche Rezeptor R₂ des erfindungsgemäßen Reagenz kann gemäß einer Ausführungsform der vorliegenden Erfindung derart ausgewählt sein, daß die Bindung von R₂ an den Analyten nicht mit der Bindung des immobilisierten Rezeptors R₁ an den Analyten interferiert. In einer anderen Ausführungsform der vorliegenden Erfindung wird der lösliche Rezeptor R₂ derart ausgewählt, daß die Bindung von R₁ an den Analyten mit der Bindung des immobilisierten Rezeptors R₁ an den Analyten interferiert. Die Auswahl des Rezeptors R₁ hängt von den jeweiligen Anforderungen an das durchzuführende Testverfahren, wie oben bereits beschrieben wurde. Der lösliche Rezeptor R₂ ist vorzugsweise ein monoklonaler Antikörper, ein monospezifischer polyklonaler Antikörper oder ein mit zwei Bindungsstellen versehenes Fragment eines derartigen Antikörpers.

Das erfindungsgemäße Reagenz ist vorzugsweise in Form eines Pulvers, einer Lösung, Suspension oder eines Lyophilisats. Das Reagenz kann weiterhin für ein immunologisches Präzipitationsverfahren erforderliche Substanzen (z.B. Hilfsstoffe, Puffer etc.) enthalten.

Ein weiterer Gegenstand der vorliegenden Erfindung ist schließlich die Verwendung der Kombination eines auf einem teilchenförmigen Trägermaterial immobilisierten Rezeptors R₁ der mit mindestens zwei unterschiedlichen Epitopen auf dem Analyten über R₁ₐ und R_{1b} spezifisch bindefähig ist und und eines löslichen, mit mindestens zwei Bindungsstellen versehenen Rezeptors R₂, wobei R₁ und R₂ beide mit einem Analyten spezifisch bindefähig sind, zur Vermeidung oder/und Verminderung des Hook-Effekts als Störung bei der Bestimmung eines Analyten durch Immunpräzipitation.

Die vorliegende Erfindung soll in Verbindung mit den Figuren 1 bis 4 weiter erläutert werden.

Es zeigen:
Figur 1 die drei Phasen der Heidelberger Kurve bei einem Immunpräzipitationstest mit einem an teilchenförmiges Trägermaterial immobilisierten Rezeptor (C.P. Price and D.H. Newman "Precipitation and Agglutination Methods" in R.F. Massayeff ed., Methods of Immunological Analysis vol. 1, 134-158, VCH Weinheim; D. Riochet "Particle Agglutination" in R.F. Massayeff ed., Methods of Immunological Analysis Vol. 1, 203-214, VCH Weinheim 1993).
Figur 2 die Wirkung eines zusätzlichen löslichen Antikörpers auf die drei Phasen der Heidelberger Kurve bei einem Immunpräzipitationstest mit einem an teilchenförmiges Trägermaterial immobilisierten Rezeptor;
Figur 3 die Wirkung des Zusatzes unterschiedlicher Konzentrationen (0 *µ*g/ml-200 *µ*g/ml) des löslichen Antikörperfragmentes F(ab')₂ <hCG>-4 auf den Hook-Effekt bei einem Immunpräzipitationstest zum Nachweis von hCG und
Figur 4 die Wirkung des Zusatzes unterschiedlicher Konzentrationen des löslichen Antikörperfragmentes F(ab')₂ <hCG>-3 auf den Hook-Effekt bei einem Immunpräzipitationstest zum Nachweis von hCG.

### Beispiel 1

Dieses Beispiel zeigt die Verwendung des erfindungsgemäßen Verfahrens auf den Nachweis von Human-Choriongonadotropin (hCG) unter Verwendung von Latexteilchen als Trägermaterial. Ein hCG-Test ist besonders für den Hook-Effekt anfällig, da ein weites Spektrum von Werten auftritt, wobei normale Konzentrationen unterhalb 10 mIU/ml liegen und pathologische Konzentrationen bis zu mehr als 10⁶ mIU/ml betragen können.

### Reagenzien

### (i) Latex:

Bei diesem Experiment wurde Streptavidin-beschichteter Latex (SA-Latex) verwendet. Die Latexteilchen hatten nach Beschichtung mit Streptavidin einen Durchmesser von etwa 180 nm. Solche Teilchen sind beispielsweise von der Firma Seradyn kommerziell erhältlich. Weiterhin ist die Herstellung von Streptavidin-beschichteten Latexteilchen in EP-A-0 464 554, Beispiel 1 beschrieben.

### (ii) Antikörper:

Es wurden vier spezifisch gegen hCG gerichtete Antikörper verwendet. Die monoklonalen Antikörper <hCG>-1 und <hCG>-2 wurden in Fab'-Fragmente überführt und biotinyliert, wobei monobiotinylierte Fab'-Fragmente erhalten wurden, die zur Beschichtung des SA-Latex verwendet wurden. Die Herstellung von Fab'-Antikörperfragmenten ist dem Fachmann wohlbekannt, beispielsweise bei Ishikawa et al., J.Immunoassay. 4 (3) 209-327 (1983) beschrieben. Die Biotinylierung von Fab-Fragmenten ist ebenfalls wohlbekannt und beispielsweise in EP-A-0 464 554, Beispiel 2 beschrieben.

Die monoklonalen Antikörper <hCG>-3 und <hCG>-4 wurden als dritter Antikörper in Lösung verwendet und wurden daher (um mögliche Störungen des Tests durch Anwesenheit der konstanten Domäne zu verhindern) in die Fab'₂-Fragmente überführt. Die monoklonalen Antikörper <hCG>-1, -3 und -4 waren gegen unterschiedliche Epitope auf der β-Untereinheit von hCG gerichtet, und der monoklonale Antikörper <hCG>-2 war gegen die α- und β-Untereinheit von hCG gerichtet.

### (iii) Puffersysteme:

a) SA-Latexpuffer: SA-Latex wurde in 200 mmol/l Glycinpuffer, pH 7,5 mit 2 % Saccharose, 0,5 % Rinderserumalbumin und 0,1 % Natriumazid gelöst.
b) Reaktionspuffer: Der Reaktionspuffer war ein 50 mmol/l Phosphatpuffer, pH 7,5, der 75 mmol/l Natriumchlorid, 0,1 % Brij, 3 % Polyethylenglykol 40000 und 0,1 % Natriumazid enthielt. Die Verwendung von Polyethylenglykol als Reaktionsverstärkungsmittel ist besonders bevorzugt, da die Reaktion in seiner Abwesenheit schlechter verläuft.
c) Standard: Die Standardlösungen wurden durch Zugabe von hCG in bekannten Konzentrationen zu Human-Serum hergestellt.

### Testprozedur:

Der Fab'-beschichtete Latex wurde durch Vermischen von SA-Latex mit den Fab'-Antikörperfragmenten <hCG>-1 und -2 im Latexpuffersystem erzeugt. Die Endkonzentration von SA-Latex im Puffer war 0,1 % (Gew./Vol.) und die der Fab'-Fragmente 0,4 *µ*g/ml. Die F(ab')₂-Antikörperfragmente <hCG>-3 und <hCG>-4 wurden in unterschiedlichen Konzentrationen dem Reaktionspuffer zugesetzt. Das Experiment wurde auf einem automatischen Hitachi 717 Analysegerät bei 37°C durchgeführt. 20 *µ*l Standardlösung wurden in eine Küvette pipettiert, wonach unmittelbar 330 *µ*l des das lösliche Antikörperfragment <hCG>-3 bzw. <hCG>-4 in unterschiedlichen Konzentrationen enthaltenden Reaktionspuffers folgten. Nach Rühren wurde das Gemisch 5 Minuten lang inkubiert, und dann wurden 50 *µ*l Latexlösung zugesetzt. Das Gemisch wurde erneut vermischt und für 5 Minuten inkubiert. Der Reaktionsverlauf wurde durch Messung der Absorptionsänderung bei 340 nm verfolgt.

### Ergebnisse:

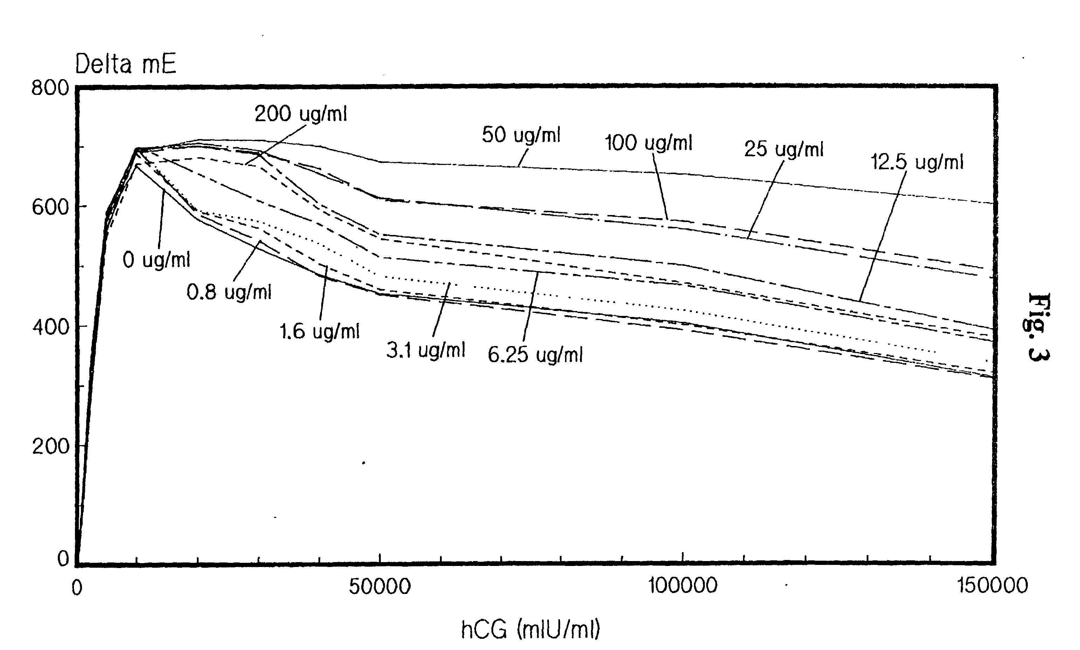

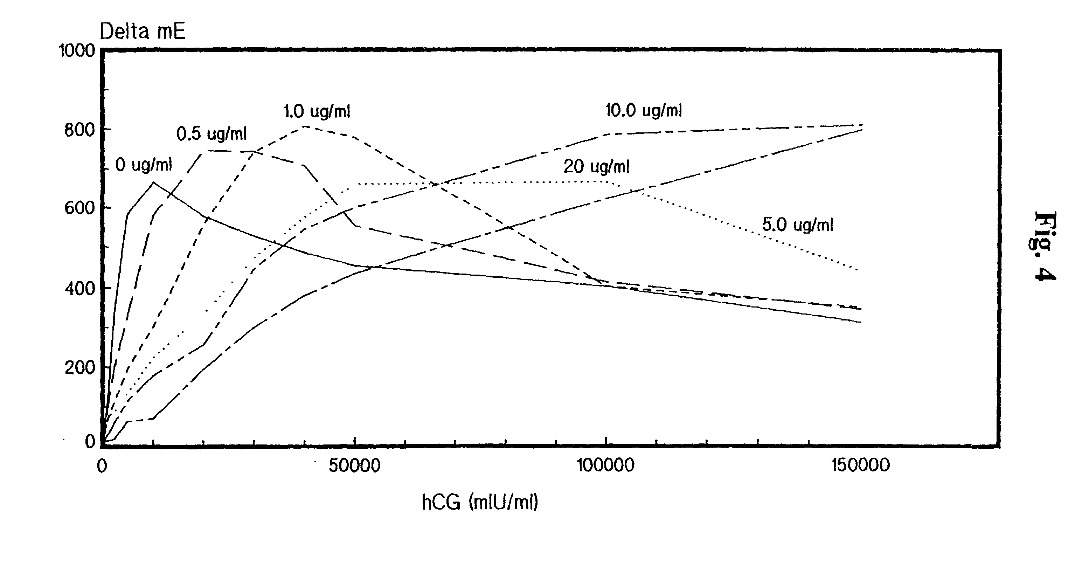

Die Figuren 3 und 4 zeigen das Ergebnis des Experiments. Aus Figur 3 ist ersichtlich, daß der Zusatz des Fab'₂-Fragments <hCG>-4 eine geringe Wirkung auf die Steigung der Kurve in der Anfangsphase besitzt, es wird jedoch eine erhebliche Verzögerung des Hook-Effekt beobachtet (siehe auch Tabelle 1). Der Zusatz des dritten Antikörpers hat in diesem Fall somit eine geringe Wirkung auf die Empfindlichkeit des Tests, führt aber zu einer ganz erheblichen Verringerung des Hook-Effekts. Die beste Wirkung wird mit einer <hCG>-4 Konzentration zwischen 25 und 100 *µ*g/ml Probeflüssigkeit gefunden.

Aus Figur 4 geht hervor, daß im Falle des Fab'₂-Fragments <hCG>-3 der Zusatz des dritten Antikörpers wiederum zu einer Verringerung des Hook-Effekts führt, in diesem Fall wird jedoch die Empfindlichkeit des Tests (d.h. die Anfangssteigung der Standardkurve) verringert (siehe auch Tabelle 2). Die Wirkung des <hCG>-3-Zusatzes tritt bereits ab einer Konzentration von 0,5 µg/ml ein.

Das unterschiedliche Verhalten der Fab'₂-Antikörperfragmente <hCG>-3 und <hCG>-4, die gegen verschiedene Epitope auf der ß-Untereinheit von hCG gerichtet sind, ist vermutlich darauf zurückzuführen, daß das von <hCG>-3 erkannte Epitop nahe bei dem von <hCG>-1 oder <hCG>-2 erkannten Epitop liegt, wodurch die Zugabe von <hCG>-3 mit der Bindung der anderen Antikörper an den Analyten interferiert, was zu einer Verringerung der Empfindlichkeit durch eine Hemmung der Bindung von <hCG>-1 oder <hCG>-2 und somit der Latexagglutinierung führt.

In den Tabellen 1 und 2 ist der Einfluß der Fab'₂-Antikörperfragmente <hCG>-4 (Tabelle 1) und <hCG>-3 (Tabelle 2) auf die Steigung der Standardkurve und das mögliche Auftreten von falschen (zu geringen) Werten bei einer hCG-Bestimmung (d.h. der Beginn des Einflusses des Hook-Effekts auf den Test) zu erkennen.

**Tabelle 1**

| Konzentration von Fab'₂ 〈hCG〉-4 (µg/ml) | Konzentration von hCG, über der das Auftreten falscher, zu geringer Werte möglich ist* (mIU/ml) | Steigerung der Standardkurve* (mE/mIU) |
|---|---|---|
| 0 | 25.833 | 0,116 |
| 0,8 | 27.500 | 0,118 |
| 1,6 | 31.666 | 0,115 |
| 3,13 | 35.416 | 0,114 |
| 6,25 | 42.500 | 0,115 |
| 12,5 | 48.584 | 0,118 |
| 25,0 | 104.583 | 0,116 |
| 50,0 | >150.000 | 0,112 |
| 100,0 | 113.333 | 0,112 |
| 200,0 | 47.500 | 0,110 |

| | | |
|---|---|---|
| * bei einer Standardkurve im Bereich 0 - 5000 mIU/ml | | |

**Tabelle 2**

| Konzentration von Fab'₂ 〈hCG〉-3 (µg/ml) | Konzentration von hCG, über der das Auftreten falscher, zu geringer Werte möglich ist* (mIU/ml) | Steigerung der Standardkurve* (mE/mIU) |
|---|---|---|
| 0 | 25.833 | 0,116 |
| 0,5 | >150.000 | 0,065 |
| 1,0 | >150.000 | 0,038 |
| 5,0 | >150.000 | 0,027 |
| 10,0 | >150.000 | 0,021 |
| 20,0 | >150.000 | 0,010 |

| | | |
|---|---|---|
| * bei einer Standardkurve im Bereich 0 - 5000 mIU/ml | | |

## Patentansprüche

1. Verfahren zur Bestimmung eines Analyten in einer Probeflüssigkeit durch Bindung des Analyten an einen mit dem Analyten spezifisch bindefähigen Rezeptor R₁, der mit mindestens zwei unterschiedlichen Epitopen auf dem Analyten über R₁ₐ und R_{1b} spezifisch bindefähig ist und der auf einem in der Probeflüssigkeit befindlichen, teilchenförmigen Trägermaterial immobilisiert ist, und Nachweis der durch Bindung des Analyten und des Rezeptors R₁ hervorgerufenen Agglutination durch nephelometrische oder turbidimetrische Methoden,
**dadurch gekennzeichnet,**
**daß** die Probeflüssigkeit weiterhin einen löslichen, mit dem Analyten spezifisch bindefähigen Rezeptor R₂ enthält, der mindestens zwei Bindungsstellen für den Analyten besitzt.

2. Verfahren nach Anspruch 1,
**dadurch gekennzeichnet,**
**daß** die Probeflüssigkeit weiterhin ein nicht-ionisches Polymer aus der Gruppe, bestehend aus Dextran mit einem Molekulargewicht von mindestens 500 kD, Polyvinylpyrrolidon mit einem Molekulargewicht von mindestens 10 kD und Polyethylenglykol mit einem Molekulargewicht von mindestens 6 kD enthält.

3. Verfahren nach Anspruch 1,
**dadurch gekennzeichnet,**
**daß** das teilchenförmige Trägermaterial aus Latexpartikeln, Metallsolen und Liposomen ausgewählt wird.

4. Verfahren nach einem der Ansprüche 1 bis 3,
**dadurch gekennzeichnet,**
**daß** man den löslichen Rezeptor R₂ derart auswählt, daß die Bindung von R₂ an den Analyten nicht mit der Bindung des immobilisierten Rezeptors R₁ an den Analyten interferiert.

5. Reagenz zur Durchführung eines Verfahrens zur Bestimmung eines bindefähigen Analyten in einer Probeflüssigkeit und Nachweis der durch Bindung des Analyten und eines Rezeptors R₁ hervorgerufenen Agglutination durch nephelometrische oder turbidimetrische Methoden,
**dadurch gekennzeichnet,**
**daß** es mindestens einen an den Analyten spezifisch bindefähigen Rezeptor R₁, der mit mindestens zwei unterschiedlichen Epitopen auf dem Analyten über R₁ₐ und R_{1b} spezifisch bindefähig ist und der auf einem teilchenförmigen Trägermaterial immobilisiert ist, und weiterhin einen mit dem Analyten spezifisch bindefähigen Rezeptor R₂, der in der Probeflüssigkeit löslich ist und mindestens zwei Bindungsstellen für den Analyten besitzt, enthält.

6. Reagenz nach Anspruch 5,
**dadurch gekennzeichnet,**
**daß** es weiterhin ein nicht-ionisches Polymer aus der Gruppe, bestehend aus Dextran mit einem Molekulargewicht von mindestens 500 kD, Polyvinylpyrrolidon mit einem Molekulargewicht von mindestens 10 kD und Polyethylenglykol mit einem Molekulargewicht von mindestens 6 kD enthält.

7. Reagenz nach Anspruch 5 oder 6,
**dadurch gekennzeichnet,**
**daß** das Trägermaterial aus der Gruppe, bestehend aus Latexpartikeln, Metallsolen und Liposomen ausgewählt ist.

8. Verwendung der Kombination eines auf einem teilchenförmigen Trägermaterial immobilisierten Rezeptor R₁, der mit mindestens zwei unterschiedlichen Epitopen auf dem Analyten über R₁ₐ und R_{1b} spezifisch bindefähig ist und eines löslichen, mit mindestens zwei Bindungsstellen versehenen Rezeptors R₂, wobei R₁ und R₂.beide mit einem Analyten spezifisch bindefähig sind, zur Vermeidung oder/und Verminderung des Hook-Effekts als Störung bei der Bestimmung eines Analyten durch Immunpräzipitation und Nachweis der durch Bindung des Analyten und des Rezeptors R₁ hervorgerufenen Agglutination durch nephelometrische und turbidimetrische Methoden.

## Claims

1. Method for the determination of an analyte in a sample liquid by binding the analyte to a receptor R₁ which is capable of specific binding to the analyte via R₁ₐ and R_{1b} to at least two different epitopes on the analyte and is immobilized on a particulate carrier material located in the sample liquid and detecting the agglutination caused by binding of the analyte and the receptor R₁ by nephelometric and turbidimetric methods,
wherein
the sample liquid additionally contains a soluble receptor R₂ which is capable of specific binding to the analyte and has at least two binding sites for the analyte.

2. Method as claimed in claim 1,
wherein
the sample liquid in addition contains a non-ionic polymer from the group comprising dextran with a molecular weight of at least 500 kD, polyvinylpyrrolidone with a molecular weight of at least 10 kD and polyethylene glycol with a molecular weight of at least 6 kD.

3. Method as claimed in claim 1,
wherein
the particulate carrier material is selected from latex particles, metal sols and liposomes.

4. Method as claimed in one of the claims 1 to 3, wherein
the soluble receptor R₂ is chosen in such a way that the binding of R₂ to the analyte does not interfere with the binding of the immobilized receptor R₁ to the analyte.

5. Reagent for carrying out a method for the determination of a bindable analyte in a sample liquid and detecting the agglutination caused by the binding of the analyte and a receptor R₁ by nephelometric or turbidimetric methods,
wherein
it contains at least one receptor R₁ which is capable of specific binding to the analyte and via R₁ₐ and R_{1b} to at least two different epitopes on the analyte and is immobilized on a particulate carrier material, and in addition contains a receptor R₂ which is capable of specific binding to the analyte, is soluble in the sample liquid and has at least two binding sites for the analyte.

6. Reagent as claimed in claim 5,
wherein
it in addition contains a non-ionic polymer from the group comprising dextran with a molecular weight of at least 500 kD, polyvinylpyrrolidone with a molecular weight of at least 10 kD and polyethylene glycol with a molecular weight of at least 6 kD.

7. Reagent as claimed in claim 5 or 6,
wherein
the carrier material is selected from the group comprising latex particles, metal sols and lipoaomes.

8. Use of the combination of a receptor R₁ which is immobilized on a particulate carrier material via R₁ₐ and R_{1b} and which is capable of specific binding to at least two different epitopes on the analyte and a soluble receptor R₂ provided with at least two binding sites, wherein
R₁ and R₂ are both capable of specific binding to an analyte, in order to avoid or/and reduce the hook effect as an interference in the determination of an analyte by immumoprecipitation and detection of the agglutination caused by binding of the analyte and the receptor R₁ by nephelometric and turbidimetric methods.

## Revendications

1. Procédé pour déterminer un analyte dans un échantillon liquide par liaison de l'analyte à un récepteur R₁ pouvant se lier spécifiquement à l'analyte qui peut se lier spécifiquement à au moins deux épitopes différents sur l'analyte par R₁ₐ et R_{1b} et qui est immobilisé sur un support particulaire situé dans l'échantillon liquide, et mise en évidence de l'agglutination due à la liaison de l'analyte et du récepteur R₁ par des méthodes néphélométriques ou turbidimétriques, **caractérisé en ce que** l'échantillon liquide contient en outre un récepteur soluble R₂ pouvant se lier spécifiquement à l'analyte, qui possède au moins deux sites de liaison pour l'analyte.

2. Procédé selon la revendication 1 **caractérisé en ce que** l'échantillon liquide contient en outre un polymère non ionique du groupe consistant en le dextrane ayant une masse moléculaire d'au moins 500 kD, la polyvinylpyrrolidone ayant une masse moléculaire d'au moins 10 kD et le polyéthylèneglycol ayant une masse moléculaire d'au moins 6 kD.

3. Procédé selon la revendication 1 **caractérisé en ce que** le support particulaire est choisi parmi les particules de latex, les sols métalliques et les liposomes.

4. Procédé selon l'une des revendications 1 à 3 **caractérisé en ce que** l'on choisit le récepteur soluble R₂ de telle manière que la liaison de R₂ à l'analyte n'interfère pas avec la liaison du récepteur R₁ immobilisé à l'analyte.

5. Réactif pour mettre en oeuvre un procédé pour déterminer un analyte pouvant se lier dans un échantillon liquide et mettre en évidence l'agglutination due à la liaison de l'analyte et d'un récepteur R₁ par des méthodes néphélométriques ou turbidimétriques, **caractérisé en ce qu'**il contient au moins un récepteur R₁ pouvant se lier spécifiquement à l'analyte, qui peut se lier spécifiquement à au moins deux épitopes différents sur l'analyte par R₁ₐ et R_{1b} et qui est immobilisé sur un support particulaire, et en outre un récepteur R₂ pouvant se lier spécifiquement à l'analyte qui est soluble dans l'échantillon liquide et qui possède au moins deux sites de liaison pour l'analyte.

6. Réactif selon la revendication 5 **caractérisé en ce qu'**il contient en outre un polymère non ionique du groupe consistant en le dextrane d'une masse moléculaire d'au moins 500 kD, la polyvinylpyrrolidone d'une masse moléculaire d'au moins 10 kD et le polyéthylèneglycol d'une masse moléculaire d'au moins 6 kD.

7. Réactif selon la revendication 5 ou 6 **caractérisé en ce que** le support est choisi dans le groupe consistant en les particules de latex, les sols métalliques et les liposomes.

8. Utilisation de la combinaison d'un récepteur R₁ immobilisé sur un support particulaire, qui peut se lier spécifiquement à au moins deux épitopes différents sur l'analyte par R₁ₐ et R_{1b}, et d'un récepteur soluble R₂ muni d'au moins deux sites de liaison, R₁ et R₂ pouvant l'un et l'autre se lier spécifiquement à l'analyte, pour éviter et/ou atténuer l'effet Hook comme perturbation lors de la détermination d'un analyte par immunoprécipitation et mise en évidence de l'agglutination due à la liaison de l'analyte et du récepteur R₁ par des méthodes néphélométriques et turbidimétriques.
